# FASCICULE DE BREVET EUROPEEN

(11) **EP 0 432 029 B1**
(45) Date de publication et mention de la délivrance du brevet: **02.03.1994**
(21) Numéro de dépôt: 90403433.7
(22) Date de dépôt: 04.12.1990
(51) Int. Cl.: C07K 7/06, A61K 37/02

(54) **Procédé de préparation de synergistines**
Verfahren zur Herstellung von Synergistinen
Process for the preparation of synergistins

(30) Priorité: 05.12.1989 FR 8916030
(43) Date de publication de la demande: 12.06.1991
(73) Titulaire: RHONE-POULENC SANTE, 92160 Antony (FR)
(72) Inventeur: Bastart, Jean-Pierre, F-77150 Lesigny (FR); Paris, Jean-Marc, F-77360 Vaires Sur Marne (FR); Radisson, Xavier, F-69006 Lyon (FR)
(74) Mandataire: Savina, Jacques

(56) Documents cités:
- EP-A- 0 135 410

## Description

La présente invention concerne la préparation de dérivés de synergistines de formule générale : 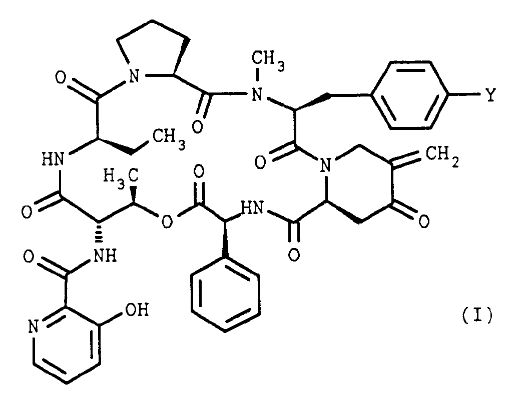
dans laquelle Y représente un atome d'hydrogène ou radical diméthylamino.

Les produits de formule générale (I) ont été décrits comme intermédiaires pour la préparation de dérivés solubles de la pristinamycine I_{A} ou de la virginiamycine, dans les brevets européens 133 097 et 248 703.

Selon l'enseignement du brevet européen 133 097, les dérivés de synergistine de formule générale (I) peuvent être obtenus à partir de pristinamycine I_{A} naturelle (Y = diméthylamino) ou de virginiamycine (Y = hydrogène) de formule générale (II) ci-après, par l'intermédiaire d'une énamine, traitée par un borohydrure alcalin en présence d'un acide organique fort.
Il a maintenant été trouvé que les dérivés de synergistine de formule générale (I) peuvent être obtenus à partir de pristinamycine I_{A} naturelle ou de virginiamycine, en passant par l'intermédiaire d'une base de Mannich de formule générale (III), dans laquelle Y est défini comme précédemment et R₁ et R₂ sont définis comme pour la formule générale (IV), puis par élimination de l'amine introduite, selon le schéma ci-après.

Il est entendu que la base de Mannich de formule générale (III) présente des formes épimères et que ses épimères et leurs mélanges entrent dans le cadre de la présente invention.

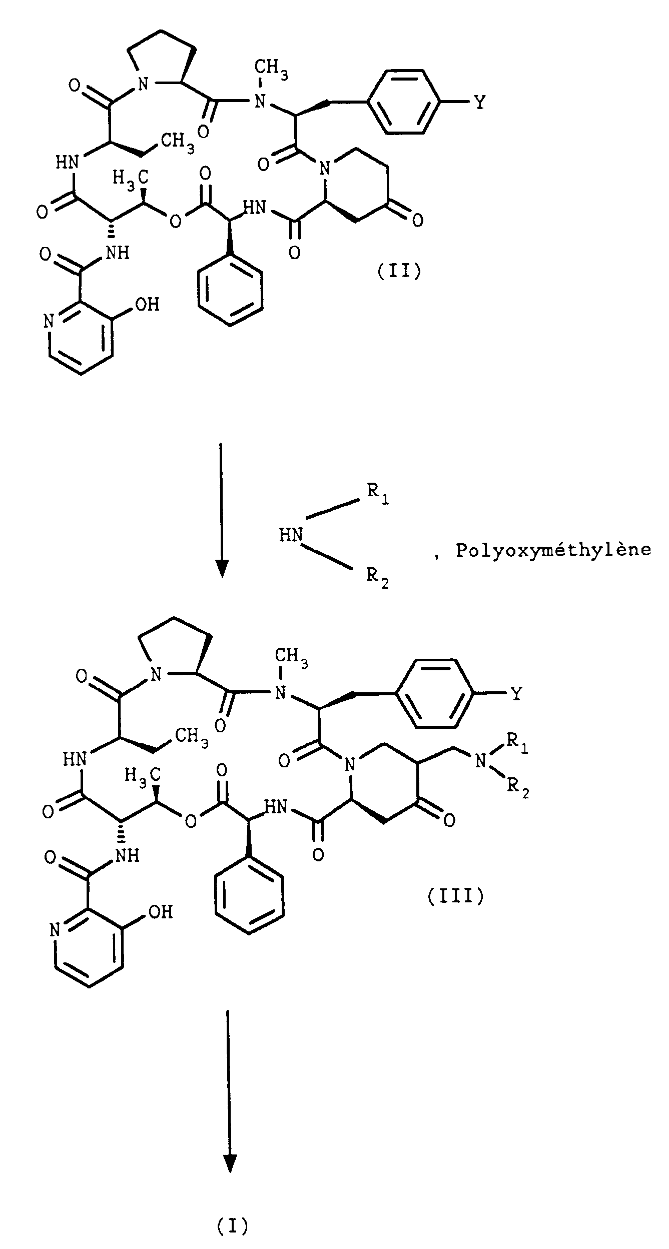

La base de Mannich est préparée en présence d'un acide, par action de polyoxyméthylène et d'une amine de formule générale : 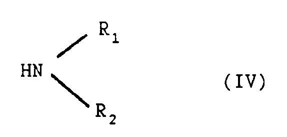
dans laquelle R₁ est un radical alcoyle droit ou ramifié contenant 1 à 3 atomes de carbone, R₂ est un radical alcoyle tel que défini ci-dessus ou un radical phényle, ou bien R₁ et R₂ forment ensemble avec l'atome d'azote auquel ils sont rattachés, un hétérocycle saturé ou insaturé contenant 5 ou 6 chaînons et éventuellement un autre hétéroatome choisi parmi l'azote ou l'oxygène.

Dans la pratique, on opère à une température comprise entre 20 et 50°C, dans un solvant protique polaire tel qu'un alcool (méthanol, éthanol, isopropanol, polyéthylèneglycol par exemple) ou dans un solvant aprotique polaire comme par exemple le diméthylsulfoxyde et l'on utilise avantageusement un large excès d'amine (10 à 40 équivalents) et de polyoxyméthylène (4 à 20 équivalents), et de préférence un léger excès d'amine par rapport à la quantité d'acide introduite, le rapport amine/acide pouvant varier de 1 à 1,3. Lorsque l'on opère en présence d'une amine de formule générale (IV) de structure cyclique, on utilise avantageusement la morpholine, la pipéridine, la pyrrolidine ou l'imidazole.

Les acides permettant de catalyser la réaction sont choisis parmi les acides forts, par exemple : les acides sulfoniques (acide méthanesulfonique, acide p.toluènesulfonique), les acides minéraux (acide sulfurique, acide chlorhydrique), les acides organiques halogénés, (acide trifluoroacétique, acide trichloroacétique). Il est également possible d'opérer dans l'acide acétique. Une autre alternative possible est l'utilisation du sel de l'amine et de l'acide employés.

De préférence l'acide sera choisi en fonction de l'amine employée. A titre d'exemple, lorsque l'on utilise la morpholine, on opère de préférence en présence d'acide méthanesulfonique ou en présence d'acide acétique ou sulfurique ; lorsque l'on utilise la pipéridine, on opère en présence d'acide acétique ou paratoluène sulfonique ; lorsque l'on utilise la diméthylamine, on opère de préférence en présence d'acide chlorhydrique.

L'élimination de l'amine est mise en oeuvre en phase hétérogène biphasique, en milieu acide tamponné, à un pH compris entre 3,5 et 5.
A titre d'exemple, on opère avantageusement en milieu acétique ou phosphorique.
La phase organique peut être choisie parmi les solvants comme les esters (acétate d'éthyle, acétate d'isopropyle par exemple), les solvants chlorés (dichlorométhane par exemple), les solvants aromatiques (toluène par exemple). On opère généralement à une température comprise entre 20 et 80°C.

Les exemples suivants donnés à titre non limitatif illustrent la présente invention.

### EXEMPLE 1

### A/ Réaction de Mannich

Dans un ballon tricol de 250 cm³, sous argon sont successivement introduits, lentement et sous agitation, 120 cm³ de méthanol, 13,8 cm³ de morpholine (0,157 mole, distillée sur potasse) et enfin 8,25 cm³ d'acide méthanesulfonique (0,125 mole, distillé), en maintenant la température du mélange à la température ambiante.
14,996 g de pristinamycine I_{A}(15,7 .10⁻³ mole, titre = 91 % en pristinamycine I_{A}) sont ajoutés en une fois, suivie de l'addition de 2,294 g de polyxyméthylène (76,3 .10⁻³ mole) et le mélange réactionnel est chauffé à 40°C, sous agitation pendant 5 heures et 30 minutes. Après retour à la température ambiante, 100 cm³ d'acétate d'éthyle sont ajoutés et le mélange est concentré sous pression réduite (à l'évaporateur rotatif) jusqu'à obtenion d'un miel (évaporation de l'azéotrope MeOH-CH₃COOEt). On ajoute de nouveau 350 cm³ d'acétate d'éthyle et 350 cm³ d'eau, puis le pH de la phase aqueuse est ramené de 6,3 à 7,5 par addition de 60 cm³ d'une solution saturée d'hydrogénocarbonate de sodium. Les phases sont séparées et la phase aqueuse est extraite 3 fois par 80 cm³ d'acétate d'éthyle. Les phases organiques réunies, sont lavées par 3 fois 100 cm³ d'eau et ces 3 dernières phases aqueuses sont extaites par 150 cm³ d'acétate d'éthyle.
L'ensemble des phases organiques est séché sur sulfate de sodium, filtré et concentré à l'évaporateur rotatif [jusqu'à obtention de 335 g de solution de base de Mannich brute (morpholinométhyl-5δ pristinamycine I_{A})].

### B/ Elimination de la morpholine

Dans un ballon tricol de 1 litre, sont successivement placés 350 cm³ d'eau, 18 cm³ d'acide acétique (18,88 g, 0,314 mole), 1,336 g d'acétate de sodium (16,3 .10⁻³ mole), puis la solution de base de Mannich est ajoutée en 5 minutes (exothermie de + 2°C). Le mélange réactionnel est chauffé à + 40°C pendant 1 heure et 50 minutes sous agitation. Après retour à température ambiante, le pH est ramené de 4 à 6,5 par addition d'une solution saturée de 250 cm³ d'hydrogènocarbonate de sodium et les phases sont séparées. La phase organique est lavée 3 fois par 100 cm³ d'eau et les phases aqueuses sont réunies et contre-extraites 3 fois par 100 cm³ d'acétate d'éthyle. Les phases organiques sont réunies et additionnées de 80 cm³ d'acétate d'éthyle pour solubiliser le produit qui commence à précipiter, puis séchées sur sulfate de sodium. Après filtration, le solvant est évaporé jusqu'à une masse totale de 116 g de la solution. Le produit cristallise. Après une nuit à + 4°C, le solide est filtré sur verre fritté (4) pour fournir 10,084 g de méthylène-5δ pristinamycine I_{A} sous forme d'un solide blanc (rendement pondéral = 72,9 %) et le filtrat est concentré à sec pour fournir encore 4,279 g de méthylène-5δ pristinamycine I_{A}(mélange des épimères) sous forme d'un solide jaune clair (rendement pondéral = 30,9 %).

### Dosage CLHP

- Cristaux : 10,08 g
   Titre en méthylène-5δ pristinamycine I_{A} : 93,5 %
   RR : 68 %
- Concentrat des jus-mères : 4,28 g
   titre en méthylène-5δ pristinamycine I_{A} : 33 %
   RR : 10 %
   Soit un RR total = 78 % en méthylène-5δ pristinamycine I_{A.}

### EXEMPLE 2

### A/ Réaction de Mannich

Dans un ballon de 25 cm³, sous argon, sont successivement introduits, lentement et sous agitation, 10 cm³ de méthanol, 0,846 cm³ de morpholine (9,7 . 10⁻³ mole, distillée sur potasse) et enfin 1,476 g d'acide p.toluènesulfonique (7,76 . 10⁻³ mole), en maintenant la température du mélange à 45°C.
1 g de pristinamycine I_{A} (0,97 . 10⁻³ mole, titre = 84 % en pristinamycine I_{A}), est ajouté en une fois. Le mélange réactionnel est additionné de 0,140 g de polyoxyméthylène (5 . 10⁻³ mole) puis chauffé à 50°C sous agitation pendant 7 heures et 45 minutes. Après retour à température ambiante, 10 cm³ d'acétate d'éthyle sont ajoutés et le mélange est concentré sous pression réduite (à l'évaporateur rotatif) jusqu'à obtention d'un miel (évaporation de l'azéotrope MeOH-CH₃COOEt). On ajoute de nouveau 30 cm³ d'acétate d'éthyle et 10 cm³ d'eau.
Les phases aqueuse et organique sont séparées et la phase aqueuse est extraite 1 fois par 10 cm³ d'acétate d'éthyle. Les phases organiques réunies sont lavées par 3 fois 10 cm³ d'eau puis concentrées à l'évaporateur rotatif (jusqu'à obtention d'environ 25 g de solution de base de Mannich brute [ morpholinométhyl-5δ pristinamycine I_{A}(mélange des épimères)].

### B/ Elimination de la morpholine

Dans un ballon de 100 cm³ sont successivement placés 25 cm³ d'eau, 0,838 cm³ d'acide acétique, 0,082 g d'acétate de sodium, puis la solution de base de Mannich est ajoutée en une fois. Le mélange réactionnel est chauffé à + 50°C pendant 1 heure sous agitation. Après retour à température ambiante, la phase aqueuse est séparée, contre-extraite par 15 cm³ d'acétate d'éthyle et les phases organiques réunies sont additionnées de 20 cm³ d'eau. Le pH est ajusté à neutralité par addition d'une solution de soude (1N) et la phase organique est séparée, lavée 2 fois par 10 cm³ d'eau et 1 fois par 10 cm³ de saumure, puis séchée sur sulfate de sodium. Après filtration, le solvant est évaporé pour obtenir 900 mg de produit sec titrant 77,5 %, soit un rendement de 81%. Ce produit pourraît être recristallisé, pour fournir la qualité habituelle avec 80 % de rendement de recristallisation.

### EXEMPLE 3

### Réaction de Mannich - morpholinométhyl-5δ pristinamycine I_{A}(mélange des épimères).

Dans un ballon tricol de 15 cm³, sous argon, sont successivement introduits, lentement et sous agitation, 1 cm³ de méthanol et 421,4 mg de méthanesulfonate de morpholine (2,30 .10⁻³ mole). 100 mg de pristinamycine I_{A} (0,115.10⁻³mole) sont ajoutés en une fois, suivie de l'addition de 69 mg de polyoxyméthylène (2,31 .10⁻³ mole) et le mélange réactionnel est agité à 28°C pendant 24 heures. Un dosage en CLHP du brut réactionnel permet de faire une estimation du rendement.
- Taux de transformation: : 98 %
- Rendement réel: : 70 %

L'élimination de la morpholine peut être effectuée dans les conditions décrites précédemment à l'exemple 1 ou à l'exemple 2.

### EXEMPLE 4

### Réaction de Mannich - morpholinométhyl-5δ pristinamycine I_{A}(mélange des épimères).

Dans un ballon tricol de 15 cm³,sous argon, sont successivement introduits, lentement et sous agitation, 3,2 cm³ de méthanol, 0,368 cm³ de morpholine (4,187 .10⁻³ mole, distillée sur potasse), et enfin 0,223 cm³ d'acide sulfurique (4,187 .10⁻³ mole, distillé), en maintenant la température du mélange à la température ambiante. 0,1 g de pristinamycine I_{A}(0,105 .10⁻³ mole, titre = 91 % en pristinamycine I_{A}) sont ajoutés en une fois, suivie de l'addition de 69 mg de polyoxyméthylène (2,31 .10⁻³ mole), et le mélange réactionnel est agité à 25°C pendant 48 heures.
Un dosage CLHP du brut réactionnel permet de faire une estimation du rendement :
- Taux de transformation: : 99 %
- Rendement réel: : 80 %

L'élimination de la morpholine peut être effectuée comme décrit précédemment à l'exemple 1 ou à l'exemple 2.

### EXEMPLE 5

### Réaction de Mannich - morpholinométhyl-5δ pristinamycine I_{A}(mélange des épimères).

Dans un ballon tricol de 15 cm³ , sous argon, sont successivement introduits, lentement et sous agitation, 3,2 cm³ de méthanol, 0,368 cm³ de morpholine (4,187 g .10⁻³ mole, distillée sur potasse) et enfin 0,251 g d'acide acétique (4,187 .10⁻³ mole) en maintenant la température du mélange à la température ambiante. 0,1 g de pristinamycine I_{A} (0,105 . 10⁻³ mole, titre = 91 % en pristinamycine I_{A}) sont ajoutés, en une fois, suivie de l'addition de 69 mg de polyoxyméthylène (2,31 .10⁻³ mole) et le mélange réactionnel est agité à 25°C pendant 24 heures.
Le dosage en CLHP du brut réactionnel, permet de faire une estimation du rendement :
- Taux de transformation: : 98 %
- Rendement réel: : 70 %

L'élimination de la morpholine peut être effectuée comme décrit précédemment à l'exemple 1 ou à l'exemple 2.

### EXEMPLE 6

A 430 cm³ de méthanol chauffé à 45°C, on ajoute, en agitant, 81,5 g de chlorhydrate de diméthylamine, 30 g de polyoxyméthylène et 43,3 g de pristinamycine I_{A.} La suspension obtenue est ensuite agitée 10 heures à 45°C Le mélange réactionnel est ensuite filtré, le gâteau est lavé avec 2 fois 25 cm³ de méthanol. Aux filtrats réunis, on ajoute 900 cm³ d'acétate d'éthyle ; le pH de la phase aqueuse est amené à 7 par addition de 65 cm³ d'une solution saturée d'hydrogénocarbonate de sodium.
La phase organique est décantée ; la phase aqueuse est extraite avec 200 cm³ d'acétate d'éthyle, puis les phases organiques réunies sont lavées avec un mélange de 500 cm³d'eau et de 50 cm³ d'une solution saturée de chlorure de sodium. La phase organique précédente contenant la diméthylaminométhyl-5δ pristinamycine I_{A} est alors versée dans une solution agitée de 43,3 cm³ d'acide acétique et de 4,3 g d'acétate de sodium dans 1000 cm³ d'eau maintenue à 35°C. Le mélange réactionnel est agité et chauffé à 35°C pendant 1 heure. La phase organique est ensuite décantée, lavée 2 fois avec un mélange de 500 cm³ d'eau et de 50 cm³ d'une solution saturée de chlorure de sodium puis, après addition de 180 cm³ de chlorure de méthylène, lavée avec 195 cm³ d'une solution saturée d'hydrogénocarbonate de sodium. La phase organique est de nouveau lavée 2 fois avec un mélange de 500 cm³ d'eau et de 50 cm³ d'une solution saturée de chlorure de sodium, puis séchée sur du sulfate de sodium, filtrée et concentrée sous pression réduite (2,7 kPa) à 30°C. Le solide obtenu est ensuite agité pendant 1 heure à une température voisine de 20°C dans 400 cm³ d'éther éthylique. La suspension obtenue est filtrée sur verre fritté, après séchage à l'air pendant une nuit à une température voisine de 20°C, on obtient 36,4 g de méthylène-5δ pristinamycine I_{A} sous forme d'une poudre blanche fondant vers 230°C. Le titre de ce produit est de 77,6 % (CLHP).

### EXEMPLE 7

### A/ Réaction de Mannich

Dans un ballon tricol de 250 cm³, sous azote, sont successivement chargés 150 cm³ de méthanol, 13,8 cm³ de morpholine (0,157 mole), puis, en 10 minutes, 8,3 cm³ d'acide méthanesulfonique (0,126 mole), et enfin 2,3707g de polyoxyméthylène (0,0785 mole) et 17,0055 g de pristinamycine I_{A} (titre 80,5 %, soit 15,78 .10⁻³ mole). Le mélange réactionnel est agité pendant 5 heures 25 minutes à 40-43°C, puis filtré sur clarcel après retour à température ambiante. Le filtrat est concentré sous pression réduite jusqu'à un poids de 75,30 g, puis additionné de 35 cm³ d'eau , 250 cm³ d'acétate d'éthyle et 100 cm³ d'eau. La phase organique est décantée et lavée par 3 fois 50 cm³ d'eau. Les phases aqueuses sont réunies et contre-extraites par 3 fois 20 cm³ d'acétate d'éthyle. Ces solutions de contre-extraction sont réunies et lavées par 20 cm³ d'eau. Les phases organiques sont réunies, séchées sur sulfate de sodium et concentrées à sec sous pression réduite pour obtenir 19,21 g de produit brut. 10,3405 g de ce produit brut sont battus dans 150 cm³ d'eau, à température ambiante, pendant 3 heures 30 minutes, puis filtrés sur verre fritté. Le solide est lavé par 3 fois 20 cm³ d'eau, puis séché pour obtenir 8,9397 g du mélange des deux épimères de morpholinométhyl-5δ pristinamycine I_{A.}
RMN ¹³C, CDCl₃, 90 MHz, HMDS (référence), δ en ppm. (numérotation indiquée selon la recommandation de J.0. Anteunis et al., Eur. J. Biochem., 58, 259 (1975) et mentionnée dans la demande de brevet européen 133 037).
Epimère majoritaire :
- 71,95: (d ; 1β)
- 67,00: (t ; -CH₂- morpholine)
- 57,6: (d ; 3α)
- 57,3: (d ; 5α)
- 56,1: (d ; 1α+6α)
- 55,4 ; 55,1: } (t ; 5ε + -CH₂-N- en 5δ)
- 53,9: (d ; 4α)
- 53,6: (t ; -CH₂- morpholine)
- 51,4: (d ; 2α)
- 47,8: (t ; 3δ)
- 45,4: (d ; 5δ)
- 41,4: (t ; 5β)
- 40,6: (q ; -N(CH₃)₂)
- 35,6: (t ; 4β)
- 31,0: (q ; -N-CH₃)
- 27,7: (t ; 3β)
- 25,1: (t ; 2β)
- 24,6: (t ; 3γ)
- 16,6: (q ; 1γ)
- 10,0: (q ; 2γ)

### B/ Elimination de la morpholine

A une solution de 4,25 cm³ d'acide acétique (74,2 .10⁻³ mole) et de 312,7 mg d'acétate de sodium (3,76 .10⁻³ mole) dans 75 cm³ d'eau est additionnée une solution dans 75 cm³ d'acétate d'éthyle de 3,9989 g de base de Mannich préparée précédemment (correspondant à environ 3,72 .10⁻³ mole). Le mélange est agité à 41-44°C pendant 4 heures 40 minutes. La réaction est terminée en 1 heure. Après retour à la température ambiante, le pH est ramené de 3,9 à 7 par addition de 60 cm³ de bicarbonate de sodium (solution aqueuse saturée). La phase aqueuse est séparée, lavée par 20 cm³ d'acétate d'éthyle et les phases organiques sont réunies, lavées par 3 fois 20 cm³ d'eau, puis séchées sur sulfate de sodium. Après concentration à sec, sous pression réduite, on obtient 3,4419 g de méthylène-5δ pristinamycine I_{A} (titre : 70 %) sous forme d'un solide blanc, avec un rendement global de 72 % à partir de la pristinamycine I_{A.}

### EXEMPLE 8

### Réaction de Mannich - morpholinométhyl-5δ pristinamycine I_{A}(mélange des épimères).

Dans un ballon tricol de 25 cm³, sont successivement introduits 5 cm³ de propanol-2, 0,40 cm³ de morpholine (4,57 .10⁻³ mole), 0,24 cm³ d'acide méthanesulfonique (3,64 .10⁻³ mole), 70,0 mg de polyoxyméthylène (2,32 .10⁻³ mole) et 501 mg de pristinamycine I_{A} (titrant 80,5%, soit 4,65 .10⁻⁴ mole). Le mélange réactionnel est agité pendant 9 heures 40 minutes à 43-45°C. Un chromatogramme CLHP de ce mélange réactionnel permet alors d'estimer un taux de transformation : TT = 92 % et un rendement en produit transformé : RT = 75 %

L'élimination de la morpholine peut être effectuée dans les conditions décrites aux exemples précédents ou à l'exemple 9.

### EXEMPLE 9

### A/ Réaction de Mannich

Dans un ballon tricol de 25 cm³ sont successivement introduits 5 cm³ d'éthanol, 0,40 cm³ de morpholine (4,57 .10⁻³ mole), 0,24 cm³ d'acide méthanesulfonique (3,64 .10⁻³ mole), 69,5 mg de polyoxyméthylène (2,31 .10⁻³ mole) et 498,6 mg de pristinamycine I_{A} (titrant : 80,5 %, soit 4,63 .10⁻⁴). Le mélange réactionnel est agité pendant 5 heures 15 minutes à 40-45°C. Après retour à température ambiante et addition de 25 cm³ de toluène et de 25 cm³ d'eau, le pH est ramené de 6 à 7 par addition d'une solution saturée de bicarbonate de sodium. La phase aqueuse est décantée et lavée par 3 fois 10 cm³ de toluène. Les phases organiques contenant la morpholinométhyl-5δ pristinamycine I_{A} (mélange des épimères), sont réunies et lavées par 3 fois 10 cm³ d'eau.

### B/ Elimination de la morpholine

La solution toluènique, de bases de Mannich brutes, obtenue précédemment est versée en une fois sur une solution de 0,53 cm³ d'acide acétique (9,26 .10⁻³ mole) et 41,4 mg d'acétate de sodium (4,98 .10⁻⁴ mole) dans 50 cm³ d'eau. Ce mélange biphasique est agité à 45-47°C pendant 3 heures 30 minutes. Après retour à la température ambiante, le pH est ramené de 4,7 à 7 par addition d'une solution aqueuse saturée de bicarbonate de sodium. La phase aqueuse est décantée et lavée par 2 fois 10 cm³ de toluène. Les phases organiques sont réunies et lavées par 2 fois 25 cm³ d'eau. Après séchage, la solution toluènique est concentrée à sec, sous pression réduite pour obtenir la méthylène-5δ pristinamycine I_{A} avec un rendement de 62 % (dosé par CLHP).

### EXEMPLE 10

### Réaction de Mannich - morpholinométhyl-5δ pristinamycine I_{A}(mélange des épimères).

Dans un ballon de 25 cm³ sont successivement introduits 5 cm³ de diméthylsulfoxyde, 0,40 cm³ de morpholine (4,57 .10⁻³ mole), 0,24 cm³ d'acide méthanesulfonique (3,64 .10⁻³ mole), 498 mg de pristinamycine I_{A} (titre 80,5 %, soit 4,62 .10⁻⁴ mole) et 72,7 mg de polyoxyméthylène (2,42 .10⁻³ mole). Le mélange réactionnel est agité pendant 4 heures 45 minutes à 43-46°C. Un dosage par CLHP permet d'estimer le taux de transformation : TT = 97 % et le rendement en produits utiles (base de Mannich contenant en outre de la méthylène-5δ pristinamycine I_{A}) par rapport au produit transformé : RT = 70%. L'élimination de la morpholine peut être effectuée comme décrit précédemment.

### EXEMPLE 11

### Réaction de Mannich - morpholinométhyl-5δ pristinamycine I_{A} (mélange des épimères).

Dans un ballon de 25 cm³ sont successivement introduits 5 cm³ d'éthylèneglycol, 0,40 cm³ de morpholine (4,57 .10⁻³ mole), 0,24 cm³ d'acide méthanesulfonique (3,64 .10⁻³ mole), 69,9 mg de polyoxyméthylène (2,32 .10⁻³ mole) et 506,8 mg de pristinamycine I_{A} (titre : 80,5 %, soit 4,7 .10⁻⁴ mole). Le mélange réactionnel est agité pendant 1 heure à environ 45°C. Un dosage par CLHP permet d'estimer le taux de transformation : TT = 95 % et le rendement en produits utiles (base de Mannich contenant en outre de la méthylène-5δ pristinamycine I_{A}) par rapport à la pristinamycine I_{A} transformée : RT = 65 %.

### EXEMPLE 12

### A/ Réaction de Mannich

Dans un ballon tricol de 25 cm³ sont successivement introduits 14 cm³ de méthanol, 1,50 cm³ de morpholine (17,1 .10⁻³ mole), 0,90 cm³ d'acide méthanesulfonique (13,6 .10⁻³ mole), 1,4032 g de virginiamycine S₁ (1,70 .10⁻³ mode) et 253 mg de polyoxyméthylène (8,4 .10⁻³ mode). Après 3 heures 30 minutes d'agitation à 45°C, la température est ramenée à environ 20°C, et le méthanol est évaporé sous pression réduite. Le résidu brut est repris par 30 cm³ d'acétate d'éthyle et 30 cm³ d'eau. le pH est ramené de 6 à 7 par addition d'une solution aqueuse saturée de bicarbonate de sodium, la phase aqueuse est décantée et lavée par 3 fois 10 cm³ d'acétate d'éthyle. Les phases organiques contenant la morpholinométhyl-5δ virginiamycine S₁ (mélange des épimères), sont réunies et lavées par 3 fois 10 cm³ d'eau.

### B/ Elimination de la morpholine

Dans un ballon rond de 250 cm³, la solution organique de base de Mannich obtenue précédemmant est versée sur une solution de 1,95 cm³ d'acide acétique (34 .10⁻³ mole) et de 149 mg d'acétate de sodium (1,79 .10⁻³ mole) dans 50 cm³. Le mélange biphasique est agité à 46°C pendant 3 heures. Après retour à la température ambiante, le pH de la phase aqueuse est ramené de 4,05 à 6,7 par addition d'une solution saturée de bicarbonate de sodium. La phase aqueuse est décantée et lavée par 3 fois 10 cm³ d'acétate d'éthyle, les phases organiques sont réunies, et l'ensemble est lavé par 3 fois 10 cm³ d'eau. Après séchage sur sulfate de sodium et filtration, la solution organique est concentrée à 15,3 g et la méthylène-5δ virginiamycine S₁ cristallise pendant une nuit à +4°C. Après filtration, on obtient ainsi 581,5 mg de produit pur (les jus mères peuvent être concentrés pour fournir un second lot de produit cristallisé de bonne pureté).

## Revendications

1. Procédé de préparation de synergistines de formule générale : 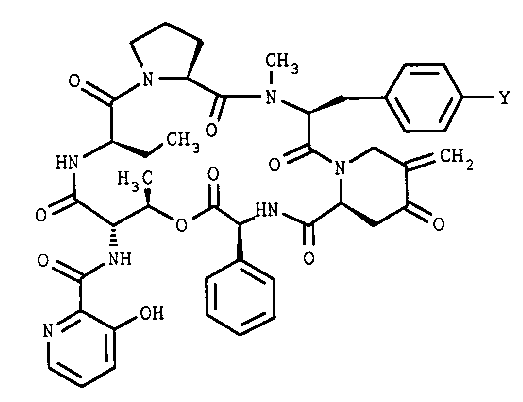 dans laquelle Y représente un atome d'hydrogène ou un radical diméthylamino, caractérisé en ce que l'on prépare une base de Mannich de formule générale : 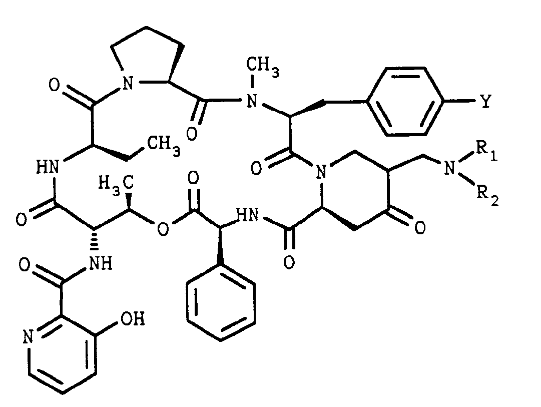 dans laquelle Y est défini comme ci-dessus et R₁ représente un radical alcoyle droit ou ramifié contenant 1 à 3 atomes de carbone, R₂ est un radical alcoyle tel que défini précédemment ou un radical phényle, ou bien R₁ et R₂ forment ensemble, avec l'atome d'azote auquel ils sont attachés, un hétérocycle saturé ou insaturé contenant 5 ou 6 chaînons et éventuellement un autre hétéroatome choisi parmi l'azote ou l'oxygène, à partir de la pristinamycine I_{A} ou de la virginiamycine, puis élimine l'amine introduite.

2. Nouveau dérivé de synergistine de formule générale : 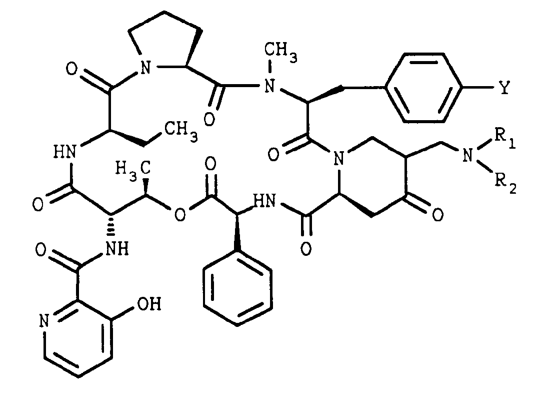 dans laquelle R₁, R₂ et Y sont définis comme dans la revendication 1, sous ses formes épimères ou leurs mélanges.

3. Utilisation d'un dérivé de synergistine selon la revendication 2, pour la préparation d'un dérivé soluble de la pristinamycine I_{A} ou de la virginiamycine.

## Patentansprüche

1. Verfahren zur Herstellung von Synergistinen der allgemeinen Formel 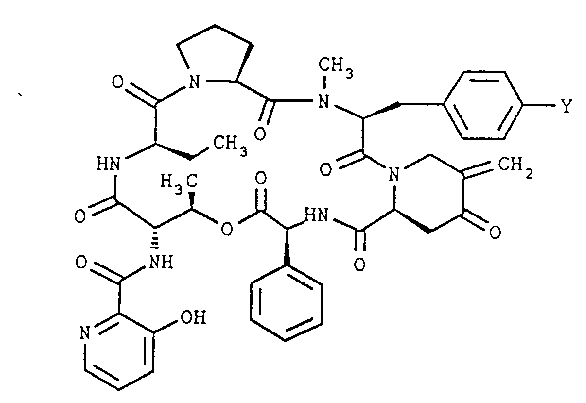 in der Y ein Wasserstoffatom oder einen Dimethylaminorest darstellt, dadurch gekennzeichnet, daß man eine Mannichbase der allgemeinen Formel 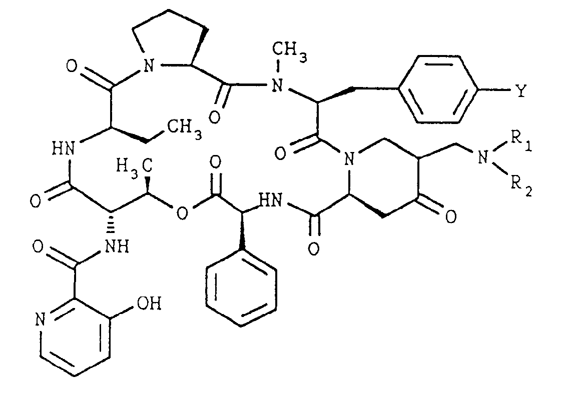 in der Y wie vorstehend definiert ist und R₁ einen geraden oder verzweigten Alkylrest mit 1 bis 3 Kohlenstoffatomen darstellt, R₂ einen wie vorstehend definierten Alkylrest oder einen Phenylrest bedeutet, oder auch R₁ und R₂ zusammen mit dem Stickstoffatom, an das sie gebunden sind, einen gesättigten oder ungesättigten Heterocyclus mit 5 oder 6 Kettengliedern und gegebenenfalls einem anderen Heteroatom, ausgewählt unter Stickstoff und Sauerstoff. bilden, ausgehend von Pristinamycin I_{A} oder Virginiamycin herstellt und anschließend das eingeführte Amin entfernt.

2. Neues Synergistinderivat der allgemeinen Formel 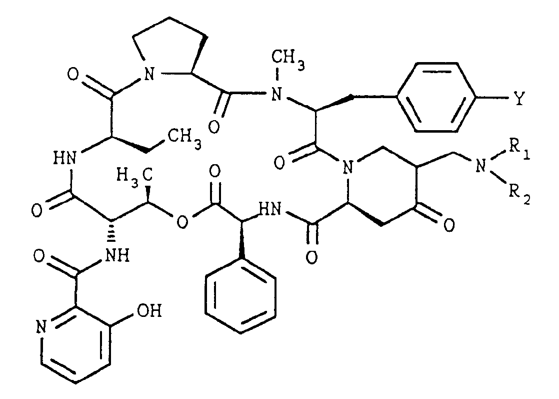 in der R₁, R₂ und Y wie in Anspruch 1 definiert sind, in seinen epimeren Formen und ihren Mischungen.

3. Verwendung eines Synergistinderivates nach Anspruch 2 zur Herstellung eines wasserlöslichen Derivates von Pristinamycin I_{A} oder Virginiamycin.

## Claims

1. Process for preparing synergistins of general formula: 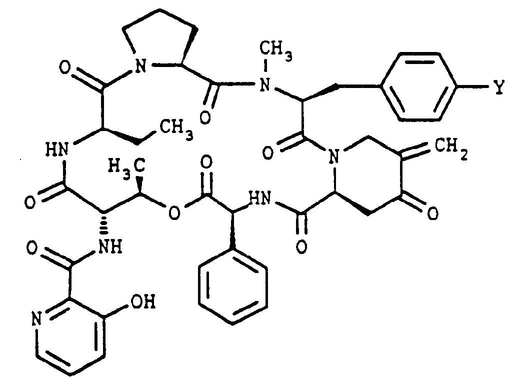 in which Y represents a hydrogen atom or a dimethylamino radical, characterized in that a Mannich base of general formula: 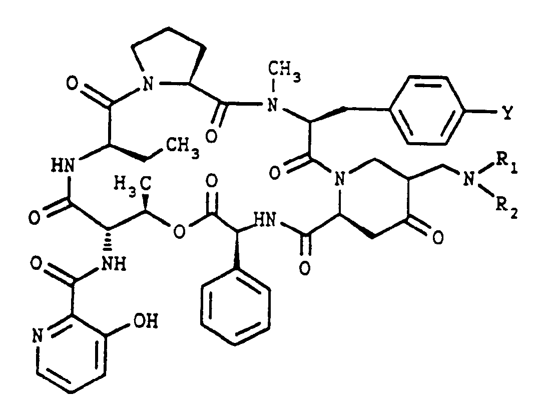 in which Y is defined as above and R₁ represents a linear or branched alkyl radical containing 1 to 3 carbon atoms, R₂ is an alkyl radical as defined above or a phenyl radical, or alternatively R₁ and R₂, together with the nitrogen atom to which they are attached, form a saturated or unsaturated 5- or 6-membered heterocycle optionally containing another hetero atom selected from nitrogen and oxygen, is prepared from pristinamycin I_{A} or from virginiamycin, and the amine introduced is then eliminated.

2. New synergistin derivative of general formula: 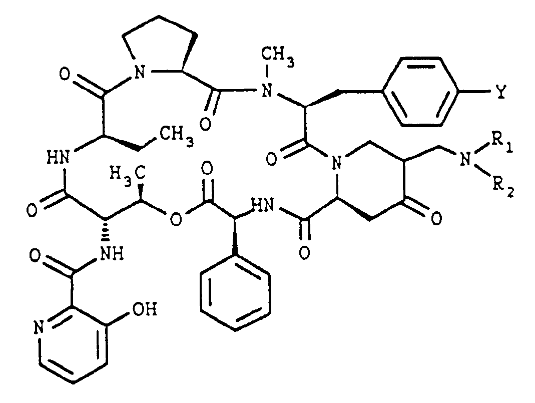 in which R₁, R₂ and Y are defined as in claim 1, in its epimeric forms or the mixtures thereof.

3. Use of a synergistin derivative according to claim 2 for the preparation of a soluble derivative of pristinamycin I_{A} or of virginiamycin.
